# EUROPEAN PATENT APPLICATION

(11) **EP 1 214 949 A2**
(43) Date of publication of application: **19.06.2002**
(21) Application number: 01310163.9
(22) Date of filing: 05.12.2001
(51) Int. Cl.: A61L 9/12, A61L 9/14

(54) **Dispensing device**

(30) Priority: 06.12.2000 GB 0029748
(71) Applicant: Dudley Industries Ltd., Lytham, Lancs. FY8 5AT (GB)
(72) Inventor: Fielding, Robert Michael Georg, St. Annes, Lancs. FY8 3HX (GB)
(74) Representative: Powell, Stephen David

(57) **Abstract**

A device for intermittently dispensing, e.g. a fragrance, incorporates a programmable microchip 17 which monitors ambient light levels and is arranged to switch on the dispensing operation in advance of the time when use of the device is anticipated. A photoelectronic panel 18 is used as a power source. The ambient light levels may be detected by the photoelectronic panel 18 or another light sensitive device. A battery 45 is also provided to power timing means. The device operates in a dispensing cycle, the duration of which may be set; after being switched off it is next switched on at the same stage of the dispensing cycle as it was last switched off.

## Description

The present invention relates to dispensing devices, and in particular to programmable dispensing devices powered by photoelectricity.

Conventional automatic dispensing devices are operated continuously, and the continuous operation leads to a number of problems. Specifically, continuously sprinkled perfume accumulates in the air in dense concentrations, which would be unpleasant for people in the room and in addition might cause them to lose their sensitivity to the scent. Moreover, continuous operation results in a waste of power and perfume. UK Patent Application GB 2337203A discloses a dispensing device which is operated by photoelectricity and which has a dispensing cycle that can be set to operate 24 hours a day, 7 days a week or from 6am to 6pm Monday to Friday only. Thus, one of two problems can arise. Either perfume is wasted by being dispensed when it is not required, or people may occupy the room before the device is switched on.

This invention seeks to overcome or reduce the above problems.

European Patent Application 0485134 discloses an air freshener dispenser which functions in response to ambient light levels. It also provides indications of when air freshener is expected to be exhausted.

According to a first aspect of the invention there is provided a device for intermittently undertaking a dispensing operation, with means for switching off the device during periods of time when dispensing is not required and means for switching on the device when dispensing is required, wherein there is provided means for detecting ambient light levels, means for storing information concerning when dispensing is required, and computing means responsive to the detecting means and the storing means to activate the switching on means at or in advance of the time when dispensing is required.

An advantage of the above arrangement is that it avoids waste of the product to be dispensed.

The device is preferably powered, at least partially, by a photoelectric panel which has the advantage of providing a cheap power supply. However, the photoelectric panel is preferably complemented by a separate electric power source, e.g. a battery, especially to operate timing means of the device. An advantage of this arrangement is that timing operations can continue even if the device is subjected to long periods of darkness.

The device may comprise a test button, which can be operated to check whether the battery needs replacing. A different type of actuation of the test button may be used to check which of two dispensing canisters of the device needs to be replaced. An advantage of using the same button for two different purposes is that it reduces the number of control elements visible from the exterior of the device.

In preferred embodiments the photoelectric panel constitutes both the means for detecting ambient light levels and a power source.

Preferably, the device operates in a predetermined cycle and may comprise means, e.g. one or more switches, for setting the length of the cycle.

Previous photosensitive dispensing devices operate in cycles depending on the required operating light level. These devices switch off when the light level falls below the threshold value. When the light rises above the operating threshold these devices switch on and start their dispensing cycle from the beginning thereby dispensing amounts of unwanted and unnecessary perfume and wasting electricity.

The present invention also seeks to overcome or reduce this problem.

According to a second aspect of the present invention there is provided a device for undertaking a dispensing operation in a predetermined cycle, means for detecting ambient light levels, means for switching off the device in response to the ambient light level falling below a corresponding threshold value, and means for switching on the device in response to the ambient light level rising above a corresponding threshold value, wherein said switching on means switches the device on at the same stage of the dispensing cycle as it was when the immediately preceding switching off operation occurred.

An embodiment of the present invention will now be described, by way of example only, with reference to the accompanying drawings of which:

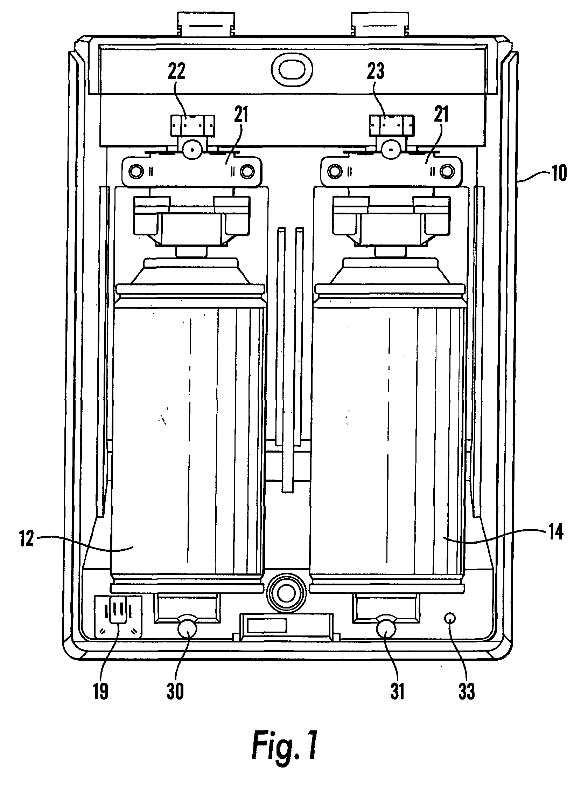

Fig 1 shows a sectional front view of the preferred dispensing unit; and

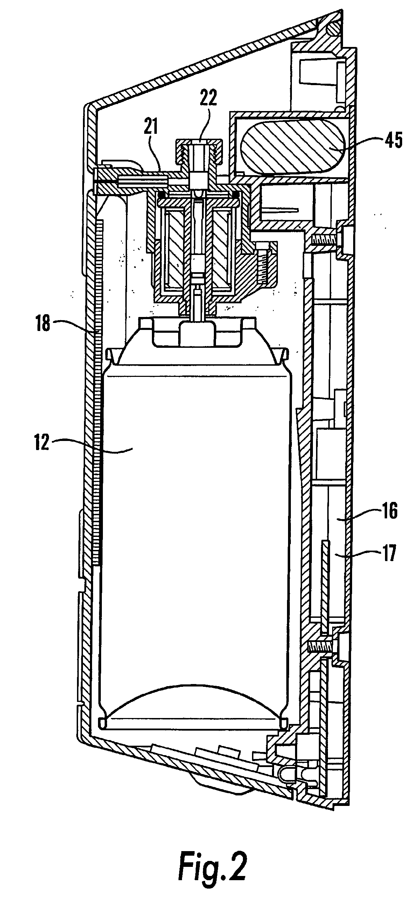

Fig 2 shows a cross-sectional side view of the preferred dispensing unit.

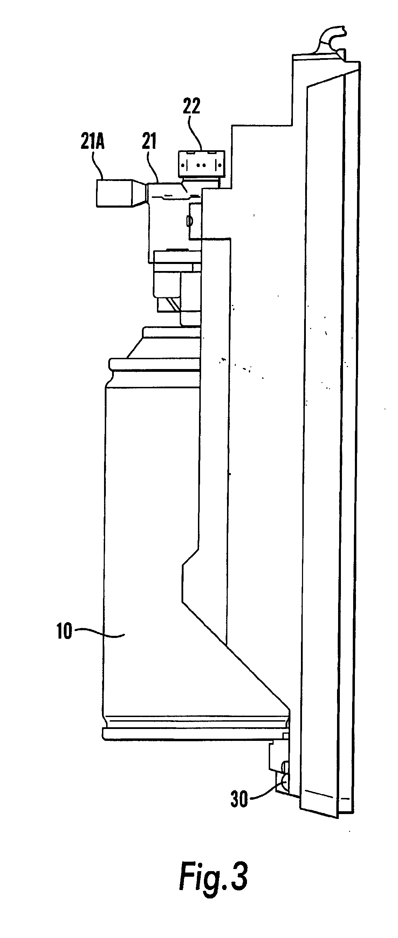

Fig 3 shows a sectional side view showing an inner casing of the preferred dispensing unit.

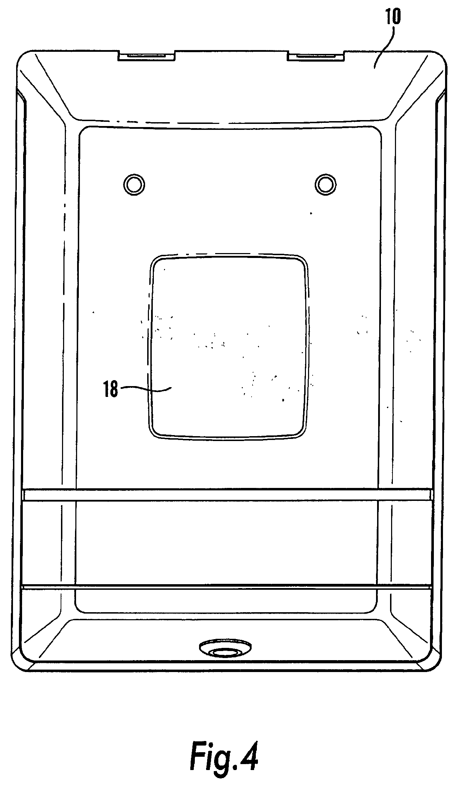

Fig 4 shows a front view of the preferred dispensing unit showing an outer casing.

Referring to the drawings, the dispensing unit comprises a casing 10, first 12 and second 14 independent pressurised canisters for containing the fragrant substances, a spray head 21, comprising a nozzle 21A for dispensing the fragrant substance, attached to each canister, two thermistor sensing canister operation devices 22, 23 mounted separately on each of the fragrant containing canisters, an electronic control circuit 16 incorporating the programmable microchip 17 and a solar panel type photoelectric cell device 18.

The circuit 16 is supplied with power generated by the photoelectric cell device 18 mounted vertically towards the front of the casing. The dispensing device also has a dil (double in line) switch timer means 19 for selecting a time delay between dispensing operations of the spray heads 21 to give delays of half, one or two hours. The dispensing device also incorporates two 'canister empty' indicator LED's 30,31, one for each canister 12,14, and a test button 33 used to cancel the LED's when an empty canister is replaced. Short operation of the test button 33 indicates which canister needs replacing, longer operation initiates a test cycle to check correct functioning of the unit and whether battery 45 needs replacing.

The programmable microchip 17 within the dispensing unit can be programmed to recognise a repetitive pattern of change from a light level below a predetermined value to a level above the predetermined value and anticipate the time of day that this would occur. This change can either be a change in the natural light level, as at the change from night to day, or a change in artificial light level, as in switching on a light in the room in which the unit is installed. The microchip controller 17 is programmed to start the function of the unit at a predetermined time, prior to the time that the change in light level, as described above, would be expected to occur.

The dispensing device is powered by the photoelectric panel 18 and contains an additional battery power source 45. The unit is light sensitive due to the photoelectric panel 18 but a similar light sensitive device e.g. a photoresistor may be used to detect the change in light levels. Using the solar panels provided, the dispensing device is set to operate normally at 100 LUX and to assume dark condition at 0.5 LUX. The timing means of the unit is powered by the additional power source. The period between the doses of the dispensing unit is set by 2 dil switches. If both these dil switches are in the off position, the period between the doses is 30 minutes. If dil switch 1 (SW1) is in the on position and dil switch 2 (SW2) is in the off position the period between doses is set to 1 hour. If dil switch 2 (SW2) is set to the on position the period between doses then becomes 2 hours and SW1 can either be on or off without affecting the dosage period.

In normal use at constant high light levels, the dispensing unit dispenses fragrance at determined intervals but when the light level falls below a predetermined level, the light sensitive photoelectric unit senses and records the time and ceases operation of its fragrant release cycles. The programmable microchip stores the exact point in the cycle the dispensing unit was in and when the light level returns to the operable level, the dispensing unit continues its cycle from where it left off when the light level fell below the predetermined operable level.

It is assumed that the equipment is used in a 7-day cycle environment. The programmable microchip monitors the first 7 days use and updates the use cycle each day after the first 7 days. The first dose of the day will be given 70 minutes before the expected start time i.e. 7 days minus 70 minutes after the dark to light signal was confirmed 7 days ago. A 7 day cycle automatically makes allowance for weekends. If the daily cycle is started and no light is seen after 3 hours then an abnormality will be assumed and dosing will stop until the next expected start of the detection of light.

The above-described arrangement has several advantages. In particular, it is useful in the case of a dispensing that has infrequent operation, for example, one hour between operations. This is an important aspect for a dispensing unit designed for use as a piece of service equipment, provided on a contract basis. This unit is also energy efficient saving needless operation due to the light dependant 'timer switch' allowing the unit to function in cycles that are cost effective and convenient. The operation of the dispensing unit in a 7 day cycle allows it to include more than 7 light/dark periods - the programme will assume these to be short days and respond as if they were normal days. In this case, there could be 14 or more 'days' in the 7-day week. The added periods could be generated by a customer cycle of morning and evening work for example.

Various modifications may be made to the above-described arrangement. For example, the microchip controller of the dispensing unit could also be programmed to carry out functions at the other end of its normal operating sequence, i.e. after the light level falls below a predetermined level for example the unit could operate after a period of time the room light was switched off or the change from day to night. The dispensing unit may also cyclically dispense a substance other than perfume, for example, water droplets to keep the moisture content in the air above a certain level in a greenhouse, insect repellent to keep the concentration of the repellent at a high enough level to be constantly effective or odour neutraliser in the case of an odourous room which constantly needed to be deodourised. The functionality of the dispensing device may be due to either the hardware or software of the device itself.

The dispensing device contains an additional battery power source but alternatively may have an electric power source as a back up. This alternative electric power source would reduce the need to replace batteries when they were spent and reduce the cost of battery replacement.

The dispensing unit may also operate entirely on photoelectricity from artificial light with no additional power source. The function of storing a 'point' in the dispensing cycle would remain provided the unit was operating in sufficient constant light for the photoelectric power source to generate the required power. The light may be fluorescent light.

The programmable microchip of the dispensing unit could also be used in a central heating unit or in a conventional heating unit to allow the heating to be turned on or off prior to a change in the light level. This would allow the user to programme the heating unit to turn on before the room where it was installed was occupied, therefore when the room became occupied, the room would already be heated. Alternatively, the heating unit with the programmable microchip may be programmed to turn the heating unit off a specified time period before the room was expected to become unoccupied.

The programmable microchip of the dispensing unit has many light dependant applications e.g. the photosensitive programmable microchip could be used in lighting applications to programme the light to activate when the light level fell below a predetermined level. This cost and energy saving device would allow the light to remain unmonitored and not waste energy and money when lit when the natural light was sufficient.

The programmable microchip can be mounted at any convenient location at the front or the rear side of the circuit board carrying control circuit 16.

## Claims

1. A device for intermittently undertaking a dispensing operation, with means for switching off the device during periods of time when dispensing is not required and means for switching on the device when dispensing is required, wherein there is provided means (18) for detecting ambient light levels, means for storing information concerning when dispensing is required, and computing means (17) responsive to the detecting means and the storing means to activate the switching on means at or in advance of the time when dispensing is required.

2. A device according to claim 1 which is powered, at least partially, by a photoelectric panel (18).

3. A device according to claim 2, comprising timing means which are powered by a separate electric power source (45).

4. A device according to claim 3, wherein the separate electric power source is a battery (45).

5. A device according to claim 4 having a test button (33), which can be operated to check whether the battery (45) needs replacing.

6. A device according to claim 5 comprising two dispensing canisters (12, 14), wherein the test button (33) can be operated to check which canister needs replacing.

7. A device according to any of claims 2 to 6 wherein said photoelectric panel (18) also constitutes the means for detecting ambient light levels.

8. A device according to any preceding claim which operates in a predetermined cycle.

9. A device according to claim 8 comprising means (19) for setting the length of said cycle.

10. A device according to claim 8 or 9 wherein the dispensing operation is switched off in response to the ambient light falling below a corresponding threshold value and is switched on in response to the ambient light level rising above a corresponding threshold value wherein said switching on means switches the device on at the same stage of the dispensing cycle as it was when the immediately preceding switching off operation occurred.
